# EUROPEAN PATENT SPECIFICATION

(11) **EP 4 486 248 B1**
(45) Date of publication and mention of the grant of the patent: **17.06.2026**
(21) Application number: 23718570.7
(22) Date of filing: 23.02.2023
(51) Int. Cl.: A61F 2/00, A61F 2/26

(54) **AN IMPLANTABLE DEVICE COMPRISING A PRESSURIZED FLUID RESERVOIR**
EIN IMPLANTIERBARES VORRICHTUNG, DAS EINEN UNTER DRUCK STEHENDEN FLÜSSIGKEITSBEHÄLTER UMFASST
UN DISPOSITIF IMPLANTABLE COMPRENANT UN RÉSERVOIR DE FLUIDE SOUS PRESSION

(30) Priority: 28.02.2022 US 202263268644 P; 22.02.2023 US 202318172933
(43) Date of publication of application: 08.01.2025
(73) Proprietor: Boston Scientific Scimed Inc., Maple Grove, Minnesota 55311-1566 (US)
(72) Inventor: WATSCHKE, Brian P., Minneapolis, Minnesota 55405 (US)
(74) Representative: Pfenning, Meinig & Partner mbB
(86) International application number: PCT/US2023/063109
(87) International publication number: WO 2023/164534

(56) References cited:
- US-A1- 2019 133 737
- US-A1- 2020 222 188
- US-B1- 6 482 145

## Description

### TECHNICAL FIELD

This disclosure relates generally to an implantable device having an electronic pump assembly that can control and regulate pressure in an inflatable member.

### BACKGROUND

An implantable device may include a pressurized container that can transfer fluid to an inflatable member. In some examples, if a patient requires less (or more) pressure in the inflatable member, a revision surgery may be required to adjust the implantable member.

An implantable device comprising a pressurized fluid reservoir configured to hold fluid and an inflatable member comprising a pressurize fluid reservoir configured to hold fluid;an inflatable member and an electronic pump assembly, including: a pump, an active valve, a pressure sensor configured to monitor a pressure of the inflatable member; and a controller configured to cause the active valve to be in an open position during at least a portion of an inflation cycle to allow the fluid to be transferred from the pressurized fluid reservoir to the inflatable member, the controller configured to transition the active valve to a closed position in response to the pressure of the inflatable member achieving a target pressure is known from the document US-A-2020/222188.

### SUMMARY

The present invention relates to an implantable device comprising a pressurized fluid reservoir configured to hold fluid and an inflatable member as set forth in the appended claims. The method of using the device does not form part of the invention. According to an aspect, an implantable device includes a pressurize fluid reservoir configured to hold fluid, an inflatable member; and an electronic pump assembly. The electronic pump assembly includes a pump, an active valve, a pressure sensor configured to monitor a pressure of the inflatable member, and a controller configured to cause the active valve to be in an open position during at least a portion of an inflation cycle to allow the fluid to be transferred from the pressurized fluid reservoir to the inflatable member. The controller is configured to transition the active valve to a closed position in response to the pressure of the inflatable member achieving a target pressure.

The implantable device may include one or more of the following features (or any combination thereof). The controller is configured to cause the pump to operate during a deflation cycle to transfer the fluid from the inflatable member to the pressurized fluid reservoir. The electronic pump assembly includes an antenna configured to receive a wireless control signal from an external device, where the controller is configured to activate the inflation cycle or the deflation cycle based on the wireless control signal. The fluid is transferred from the pressurized fluid reservoir to the inflatable member during the inflation cycle without using the pump. The active valve is disposed in parallel with the pump. The controller is configured to control the pump to operate to transfer a portion of the fluid from the inflatable member to the pressurized fluid reservoir in response to the pressure of the inflatable member being greater than the target pressure. The controller is configured to transition the active valve from the open position to a partially-opened position in response to the pressure exceeding a threshold level, the threshold level being a value that is less than the target pressure. The electronic pump assembly may include a first pressure sensor to monitor the pressure of the inflatable member and a second pressure sensor configured to monitor the pressure of the pressurized fluid reservoir. The controller is configured to cause the active valve to be in the open position for a period of time in response to the pressure of the pressurized fluid reservoir exceeding a threshold level. The controller is configured to detect a posture of a patient of the implantable device based on pressure readings from at least one of the first pressure sensor or the second pressure sensor, the controller configured to adjust the pressure of the inflatable member based on the detected posture.

According to an aspect, an implantable device includes a pressurize fluid reservoir configured to hold fluid, an inflatable member, and an electronic pump assembly, including a pump, an active valve, a pressure sensor configured to monitor a pressure of the inflatable member, and a controller configured to cause the active valve to be in an open position until the pressure of the inflatable member achieves a target pressure during at least a portion of an inflation cycle.

The implantable device may include one or more of the above/below features (or any combination thereof). The controller is configured to cause the pump to operate during a deflation cycle to transfer the fluid from the inflatable member to the pressurized fluid reservoir. The electronic pump assembly includes an antenna configured to receive a wireless control signal from an external device, where the controller is configured to activate the inflation cycle or the deflation cycle based on the wireless control signal. The active valve is disposed in parallel with the pump. The controller is configured to control the pump to operate to transfer a portion of the fluid from the inflatable member to the pressurized fluid reservoir in response to the pressure of the inflatable member being greater than the target pressure. The controller is configured to transition the active valve from the open position to a partially-opened position in response to the pressure exceeding a threshold level, the threshold level being a value that is less than the target pressure. The pressure sensor is a first pressure sensor, and the electronic pump assembly includes a second pressure sensor configured to monitor a pressure of the pressurized fluid reservoir. The controller is configured to cause the active valve to be in the open position for a period of time in response to the pressure of the pressurized fluid reservoir exceeding a threshold level.

According to an aspect, a method of operating an implantable device includes receiving, by an antenna of an electronic pump assembly, a wireless control signal from an external device, generating, by a controller, a first control signal to control an active valve of the electronic pump assembly in response to the wireless control signal, and actuating, in response to the first control signal, the active valve to an open position to transfer fluid from a pressurized fluid reservoir to an inflatable member until the pressure in the inflatable member reaches a threshold level. In some examples, the method includes generating, by the controller, a second control signal to control a pump of the electronic pump assembly and actuating, in response to the second control signal, the pump to transfer the fluid from the inflatable member to the pressurized fluid reservoir. In some examples, the method includes detecting, by the controller, that the pressure of the inflatable member exceeds the threshold level, wherein the second control signal is generated in response to the pressure of the inflatable member being detected as exceeding the threshold level.

### BRIEF DESCRIPTION OF THE DRAWINGS

FIG. 1 illustrates an implantable device according to an aspect.
FIG. 2 illustrates an implantable device according to another aspect.
FIG. 3 illustrates an implantable device according to another aspect.
FIG. 4 illustrates an implantable device according to another aspect.
FIG. 5 illustrates a flow chart depicting example operations of controlling an implantable device according to an aspect.
FIG. 6 illustrates a urinary control device according to an aspect.
FIG. 7 illustrates an inflatable penile prosthesis according to an aspect.

### DETAILED DESCRIPTION

An implantable device (e.g., an artificial urinary sphincter device, an inflatable penile prosthesis) includes a fluid reservoir, an inflatable member (e.g., inflatable cuff member, inflatable cylinder(s)), and an electronic pump assembly configured to control the pressure in the inflatable member. The electronic pump assembly includes a controller, one or more active valves, one or more pumps, and one or more pressure sensors. The electronic pump assembly may include circuit board(s), batteries, and communication module(s) to control the implantable device to deliver appropriate pressure to the inflatable member.

Pressure in the inflatable member is generated by the fluid pressurized reservoir. According to the present invention the fluid is transferred from the fluid reservoir to the inflatable member without the use of a pump, as the pressure differential between the pressurized reservoir and the inflatable member will cause the fluid to flow from the pressurized reservoir to the inflatable member.

Without the electronic implementation, the pressure in the inflatable member may equilibrate with the pressurized reservoir. However, for the system to be adjustable, the pressure in the pressurized reservoir may be required to be at least equal (or greater than) to the desired pressure in the inflatable member. The electronic pump assembly is configured to sense and control the pressure in the inflatable member.

To inflate the inflatable member, the controller of the electronic pump assembly may cause an active valve to transition to an open position (in which fluid can flow through the active valve). For example, when the active valve is in the open position, the pressure in the fluid reservoir will cause the fluid to flow to the inflatable member. The controller may receive pressure readings from a pressure sensor configured to monitor a pressure in the inflatable member. Using the pressure readings, the controller may determine that the pressure in the inflatable member has reached a target (or desired pressure) and may cause the active valve to transition to a closed position (in which fluid is blocked from flowing through the active valve) so that pressure can be maintained in the inflatable member.

After the active valve is closed, the controller may determine that the pressure in the inflatable member is greater than the target pressure. For example, the time required to process the pressure readings, generate instructions, and to close the active valve may cause the pressure in the inflatable member to exceed the target pressure. In some examples, the controller may cause a pump to actuate to pump a small amount of fluid from the inflatable member to the fluid reservoir so that the pressure in the inflatable member can reach the target pressure. In some examples, the controller may determine the amount of overshoot pressure (e.g., the difference between the pressure currently in the inflatable member and the target pressure) and control a pumping rate of the pump based on the amount of overshoot pressure. For example, if the overshoot pressure is greater than a threshold amount, the controller may cause the pump to pump the fluid at a first rate (e.g., a higher rate). If the overshoot pressure is less than a threshold amount, the controller may cause the pump to pump the fluid at a second rate (e.g., a lower rate).

In some examples, the active valve is a variable controlled valve. When there is a large pressure differential between the target pressure and the actual pressure, the controller may control the active valve to be fully opened. As the controller detects the actual pressure in the inflatable member becoming close to the target pressure, the controller may control the active valve to be in a partially-opened position such that the rate at which fluid is delivered to the inflatable member is decreased, which may prevent or reduce an overpressure event. Also, by closing the active valve partially, it may take less time to fully close the active valve, which may also aid in system response time.

In some examples, the implantable device includes a pressure sensor configured to monitor a pressure of the fluid reservoir. For example, during certain activities engaged by the patient (and the posture of the patient), the pressure in the fluid reservoir may fluctuate. For example, during a period of high activity (e.g., a hard workout) or when the patient is coughing, intra-abdominal pressure may rise, which can lead to incontinence in patients. The pressure spike may be transferred to the fluid reservoir. In some examples, if the pressure readings from the pressure sensor that monitors the pressure of the fluid reservoir is greater than a threshold level, the controller may cause the active valve to transition to the open position so that at least some fluid is transferred to the inflatable member to prevent incontinence caused by increased intra-abdominal pressure.

In some examples, posture may have an effect on intra-abdominal pressure, where standing up may generate a greater pressure than lying down. In some examples, the controller is configured to detect a posture of the patient based on the pressure readings from a first pressure sensor that monitors the pressure of the fluid reservoir and/or the pressure readings from the pressure sensor that monitors the pressure of the inflatable member. In some examples, if the detected posture of the patient is a position in which the patient is lying down, the controller may reduce the pressure in the inflatable member by activating the pump to transfer a portion of the fluid from the inflatable member to the fluid reservoir. If the detected posture of the patient is a position in which the patient is standing up, the controller may increase the pressure in the inflatable member by transitioning the active valve to an open position. In some examples, the controller may detect a change in posture (e.g., laying down to standing up) based on the pressure readings, and the controller may adjust the pressure in the inflatable member in response to the change in posture. These and other features are further explained with reference to the figures.

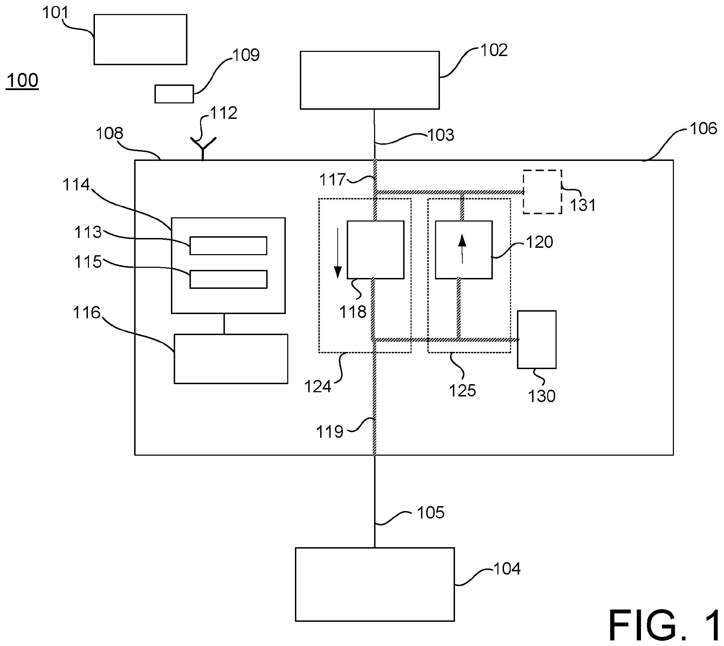

FIG. 1 illustrates an implantable device 100 according to an aspect. In some examples, the implantable device 100 is an artificial urinary sphincter device. In some examples, the implantable device 100 is an inflatable penile prosthesis. However, the implantable device 100 may include any type of medical device that transfers fluid between components of the implantable device 100.

The implantable device 100 includes a fluid reservoir 102, an inflatable member 104, and an electronic pump assembly 106 configured to transfer fluid between the fluid reservoir 102 and the inflatable member 104. In some examples, the inflatable member 104 is an inflatable cuff member configured to be implemented around a urethra of a patient. In some examples, the inflatable member 104 is a penile inflation member (e.g., one or more inflatable cylinders) that may be implanted into the corpus cavernosum of the user. The fluid reservoir 102 may be implanted in the abdomen or pelvic cavity of the user (e.g., the fluid reservoir 102 may be implanted in the lower portion of the user's abdominal cavity or the upper portion of the user's pelvic cavity). In some examples, at least a portion of the electronic pump assembly 106 may be implemented in the patient's body.

The inflatable member 104 may be capable of expanding upon the injection of fluid into a cavity of the inflatable member 104. If implanted around the urethra, the expansion of the inflatable member 104 causes the urethra to become restricted, thereby reducing the risk of incontinence in patients. For example, the electronic pump assembly 106 is configured to move fluid to pressure the inflatable cuff (e.g., the inflatable member 104), which constricts the urethra, thereby restricting the flow of urine. To urinate, the patient may operate the electronic pump assembly 106 to depressurize the inflatable cuff by transferring fluid from the inflatable cuff to the fluid reservoir 102. If implanted into the corpus cavemosum, upon injection of the fluid into the inflatable member 104, the inflatable member 104 may increase its length and/or width, as well as increase its rigidity.

The fluid reservoir 102 may include a container having an internal chamber configured to hold or house fluid that is used to inflate the inflatable member 104. According to the present invention the fluid reservoir 102 is pressurized. In some examples, the fluid reservoir 102 is a pressurized balloon. In some examples, the implantable device 100 includes a single pressurized balloon. In some examples, the implantable device 100 includes two or more pressurized balloons. The pressure in the inflatable member 104 is generated by the fluid reservoir 102. For example, the fluid can be transferred to the inflatable member 104 without the use of a pump. The pressure differential between the inflatable member 104 and the fluid reservoir 102 will cause the fluid to flow from the fluid reservoir 102 to the inflatable member 104. According to the present invention the pressure in the fluid reservoir 102 is greater than the pressure in the inflatable member 104 (e.g., even when the inflatable member 104 is at its target or maximum pressure). In some examples, the pressure in the fluid reservoir 102 is always greater than the pressure in the inflatable member 104.

The volumetric capacity of the fluid reservoir 102 may vary depending on the size of the implantable device 100. In some examples, the volumetric capacity of the fluid reservoir 102 may be 3 to 150 cubic centimeters. In some examples, the fluid reservoir 102 is constructed from the same material as the inflatable member 104. In other examples, the fluid reservoir 102 is constructed from a different material than the inflatable member 104. In some examples, the fluid reservoir 102 contains a larger volume of fluid than the inflatable member 104.

The implantable device 100 may include a first conduit connector 103 and a second conduit connector 105. Each of the first conduit connector 103 and the second conduit connector 105 may define a lumen configured to transfer the fluid to and from the electronic pump assembly 106. The first conduit connector 103 may be coupled to the electronic pump assembly 106 and the fluid reservoir 102 such that fluid can be transferred between the electronic pump assembly 106 and the fluid reservoir 102 via the first conduit connector 103. For example, the first conduit connector 103 may define a first lumen configured to transfer fluid between the electronic pump assembly 106 and the fluid reservoir 102. The first conduit connector 103 may include a single or multiple tube members for transferring the fluid between the electronic pump assembly 106 and the fluid reservoir 102.

The second conduit connector 105 may be coupled to the pump assembly 106 and the inflatable member 104 such that fluid can be transferred between the electronic pump assembly 106 and the inflatable member 104 via the second conduit connector 105. For example, the second conduit connector 105 may define a second lumen configured to transfer fluid between the electronic pump assembly 106 and the inflatable member 104. The second conduit connector 105 may include a single or multiple tube members for transferring the fluid between the electronic pump assembly 106 and the inflatable member 104. In some examples, the first conduit connector 103 and the second conduit connector 105 may include a silicone rubber material. In some examples, the electronic pump assembly 106 may be directly connected to the fluid reservoir 102.

The electronic pump assembly 106 that can monitor control and regulate the pressure within an inflatable member 104. The electronic pump assembly 106 may automatically transfer fluid between the fluid reservoir 102 and the inflatable member 104 without the user manually operating a pump (e.g., squeezing and releasing a pump bulb). The electronic pump assembly 106 may include a pump 120 (or multiple pumps 120), an active valve 118 (or multiple active valves 118), and a pressure sensor 130. In some examples, the pressure sensor 130 is configured to monitor the pressure of the fluid reservoir 102. In some examples, the electronic pump assembly 106 includes a pressure sensor 131 configured to monitor the pressure of the fluid reservoir 102. The controller 114 may control the pump 120 to pump fluid from the inflatable member 104 to the fluid reservoir 102 and control the active valve 118 to transition between an open position and a closed position. In some examples, the controller 114 may control the active valve 118 to an intermediate position (e.g., between the open position and the closed position) in which the active valve 118 has a partially-opened state that allows the fluid to flow through the active valve 118 at a rate less than the rate of the open position.

The electronic pump assembly 106 may include a battery 116 configured to provide power to the controller 114 and other components on the electronic pump assembly 106. In some examples, the battery 116 is a non-rechargeable battery. In some examples, the battery 116 is a rechargeable battery. In some examples, the electronic pump assembly 106 (or a portion thereof) (or the controller 114) is configured to be connected to an external charger to charge the battery 116. In some examples, the electronic pump assembly 106 may define a charging interface that is configured to connect to the external charger. In some examples, the charging interface includes a universal serial bus (USB) interface configured to receive a USB charger. In some examples, the charging technology may be electromagnetic or Piezoelectric.

The electronic pump assembly 106 may include an antenna 112 configured to wirelessly transmit (and receive) wireless signals 109 from an external device 101. The external device 101 may be any type of component that can communicate with the electronic pump assembly 106. The external device 101 may be a computer, smartphone, tablet, pendant, key fob, etc. A user may use the external device 101 to control the implantable device 100. In some examples, the user may use the external device 101 to inflate or deflate the inflatable member 104.

For example, in response to the user activating an inflation cycle using the external device 101 (e.g., selecting a user control on the external device 101), the external device 101 may transmit a wireless signal 109 to the electronic pump assembly 106 to initiate the inflation cycle (received via the antenna 112), where the controller 114 may control the active valve 118 to be in an open position, thereby allowing the transfer of fluid from the fluid reservoir 102 to the inflatable member 104 until a target pressure of the inflatable member 104 is reached. For example, the controller 114 may receive pressure readings from the pressure sensor 130, and, if the pressure of the inflatable member 104 has reached (or exceeded) the target pressure, the controller 114 may control the active valve 118 to be in the closed position, thereby maintaining the pressure in the inflatable member 104.

In some examples, in response to the user activating a deflation cycle using the external device 101 (e.g., selecting a user control on the external device 101), the external device 101 may transmit a wireless signal 109 to the electronic pump assembly 106 to initiate the deflation cycle (received via the antenna 112), where the controller 114 may control the pump 120 to pump fluid from the inflatable member 104 to the fluid reservoir 102. In some examples, during the deflation cycle, the active valve 118 may continue to be in the closed state. The controller 114 may control the pump 120 to continue to pump the fluid from the inflatable member 104 to the fluid reservoir 102 until a pressure in the inflatable member 104 reaches a lower threshold (e.g., zero pounds per square inch (PSI)). For example, the controller 114 may receive the pressure readings from the pressure sensor 130, and, in response to the detection of the pressure in the inflatable member 104 achieving the lower threshold, the controller 114 may control the pump 120 to deactivate (e.g., stop pumping).

The controller 114 may be any type of controller configured to control operations of the pump 120 and the active valve 118. In some examples, the controller 114 is a microcontroller. In some examples, the controller 114 includes one or more drivers configured to drive the pump 120 and the active valve 118. In some examples, the driver(s) are components separate from the controller 114. The controller 114 may be communicatively coupled to the active valve 118, the pump 120, the pressure sensor 130, and the pressure sensor 131. In some examples, the controller 114 is connected to the active valve 118, the pump 120, the pressure sensor 130, and the pressure sensor 131 via wired data lines. The controller 114 may include a processor 113 and a memory device 115. The processor 113 may be formed in a substrate configured to execute one or more machine executable instructions or pieces of software, firmware, or a combination thereof. The processor 113 can be semiconductor-based - that is, the processors can include semiconductor material that can perform digital logic. The memory device 115 may store information in a format that can be read and/or executed by the processor 113. The memory device 115 may store executable instructions that when executed by the processor 113 cause the processor 113 to perform certain operations discussed herein. The controller 114 may receive data via the pressure sensor 130, the pressure sensor 131, and/or the external device 101, and control the active valve 118 and/or the pump 120 by transmitting control signals to the active valve 118 and/or the pump 120.

The memory device 115 may store control parameters that can be set or modified by the user and/or physician using the external device 101. In some examples, the control parameters may include a target inflation pressure (e.g., target pressure) and/or a partial inflation pressure. In some examples, the target pressure is a maximum (or desired) pressure allowable in the inflatable member 104. In some examples, the partial inflation pressure is a pressure threshold when the inflatable member 104 is deflated. In some examples, the partial inflation pressure is zero PSI. In some examples, the partial inflation pressure is a value above zero PSI but lower than the target inflation pressure. A user or physician may update the control parameters using the external device 101, which can be communicated to the controller 114 via the antenna 112 and then updated in the memory device 115.

The external device 101 may communicate with the electronic pump assembly 106 over a network. In some examples, the network includes a short-range wireless network such as near field communication (NFC), Bluetooth, or infrared communication. In some examples, the network may include the Internet (e.g., Wi-Fi) and/or other types of data networks, such as a local area network (LAN), a wide area network (WAN), a cellular network, satellite network, or other types of data networks.

In some examples, the electronic pump assembly 106 includes a single pump 120. The pump 120 may be disposed in parallel with the active valve 118. In some examples, the electronic pump assembly 106 includes multiple pumps 120. In some examples, one or more pumps 120 may be disposed in parallel with each other (e.g., parallel pumps). In some examples, one or more pumps 120 may be disposed in series with each other. In some examples, the electronic pump assembly 106 includes one or more parallel pumps, one or more serial pumps, or a combination of parallel pump(s) and serial pump(s). In some examples, the pump 120 is disposed in a fluid passageway 125 that is used to empty the inflatable member 104 (e.g., during the deflation cycle).

The pump 120 is an electronically-controlled pump. The pump 120 may be electronically-controlled by the controller 114. For example, the pump 120 may be connected to the controller 114 and may receive a signal to actuate the pump 120. A pump 120 may be unidirectional in which the pump 120 can transfer fluid from the inflatable member 104 to the fluid reservoir 102. In some examples, the pump 120 is bidirectional in which the pump 120 can transfer fluid from the fluid reservoir 102 to the inflatable member 104 and from the inflatable member 104 to the fluid reservoir 102.

In some examples, the pump 120 is an electromagnetic pump that moves the fluid from the inflatable member 104 to the fluid reservoir 102 using electromagnetism. With respect to an electromagnetic pump, a magnetic fluid is set at angles to the direction the fluid moves in, and a current is passed through it.

In some examples, the pump 120 is a piezoelectric pump. In some examples, a piezoelectric pump may be a diaphragm micropump that uses actuation of a diaphragm to drive a fluid. In some examples, a piezoelectric pump may include one or more piezo pumps (e.g., piezo elements), which may be implemented by a substrate layer (e.g., a single substrate layer) of high-voltage piezo elements or may be implemented by multiple substrate layers (e.g., stacked substrate layers) of low-voltage piezo elements. In some examples, the pump 120 includes a plurality of micro-pumps (e.g., piezoelectrically-driven micro-pumps) disposed on one or more substrates (e.g., wafer(s)). In some examples, the micro-pumps include a silicon-based material. In some examples, the micro-pumps include a metal (e.g., steel) based material. In some examples, the pump 120 is non-mechanical (e.g., without moving parts).

The active valve 118 may be an electronically-controlled valve. The active valve 118 may be electronically-controlled by the controller 114. In some examples, the active valve 118 is a piezo-electric valve. In some examples, the active valve 118 is a piezo-electric diaphragm valve. The active valve 118 may be connected to the controller 114 and may receive a signal to transition the active valve 118 between the open position and the closed position. In some examples, the active valve 118 may be actuated to an intermediate position between the open position and the closed position in which the active valve 118 is partially open. When the active valve 118 is in the partially open position, fluid flows through the valve at a rate that is less than the fluid transfer rate of a fully opened valve.

In some examples, the active valve 118 is disposed in a fluid passageway 124 that is used to fill the inflatable member 104 (e.g., in the inflation cycle). In some examples, the active valve 118 may transition to the closed position to hold (e.g., substantially hold) the pressure in the inflatable member 104. In some examples, the active valve 118 may transition to the open position to transfer fluid back to the inflatable member 104.

In some examples, the electronic pump assembly 106 includes a single active valve 118. In some examples, the electronic pump assembly 106 includes multiple active valves 118. In some examples, one or more additional active valves 118 may be in series with the pump 120. In some examples, an additional active valve 118 (e.g., a series active valve 118) may be disposed in a fluid pathway portion 117 that is connected to the fluid reservoir 102. In some examples, an additional active valve 118 (e.g., a series active valve 118) may be disposed in a fluid pathway portion 119 that is connected to the inflatable member 104. These additional active valves 118 may reduce leakage when at the target pressure.

The pressure sensor 130 and the pressure sensor 131 are communicatively coupled to the controller 114 such that the controller 114 can receive signals (e.g., pressure readings) from the pressure sensor 130. In some examples, the pressure sensor 130 is disposed between the pump 120 and the inflatable member 104, as shown in FIG. 1. In some examples, the pressure sensor 131 is disposed between the pump 120 and the fluid reservoir 102. The pressure sensor 130 may measure the pressure in the inflatable member 104, and, in some examples, the pressure sensor 131 may measure the pressure of the fluid reservoir 102. In some examples, the electronic pump assembly 106 may include additional pressure sensors, which can be located at various positions in the electronic pump assembly 106.

The controller 114 may receive the measured pressure from the pressure sensor 130 and/or the pressure sensor 131 and automatically control the active valve 118 and/or pump 120 to regulate the pressure. In some examples, after the active valve 118 is closed (at the end of an inflation cycle), the controller 114 may determine that the pressure in the inflatable member 104 is greater than the target pressure. For example, the time required to process the pressure readings, generate instructions, and to close the active valve 118 may cause the pressure in the inflatable member 104 to exceed the target pressure. In some examples, the controller 114 may cause the pump 120 to actuate to transfer a small amount of fluid from the inflatable member 104 to the fluid reservoir 102 so that the pressure in the inflatable member 104 can reach the target pressure.

In some examples, the controller 114 may determine the amount of overshoot pressure (e.g., the difference between the pressure currently in the inflatable member 104 and the target pressure) and control a pumping rate of the pump 120 based on the amount of overshoot pressure. For example, if the overshoot pressure is greater than a threshold amount, the controller 114 may cause the pump 120 to transfer the fluid at a first rate (e.g., a higher rate). If the overshoot pressure is less than a threshold amount, the controller 114 may cause the pump to transfer the fluid at a second rate (e.g., a lower rate). In some examples, the second rate is less than the first rate.

In some examples, the active valve 118 is a variable controlled valve. When there is a large pressure differential between the target pressure and the actual pressure, the controller 114 may control the active valve 118 to be fully opened. As the controller 114 detects the actual pressure in the inflatable member 104 becoming close to the target pressure (e.g., exceeding a threshold level that is less than the target pressure), the controller 114 may control the active valve 118 to be in a partially-opened position such that the rate at which fluid is delivered to the inflatable member 104 is decreased, which may prevent or reduce an overpressure event. Also, by closing the active valve 118 partially, it may take less time to fully close the active valve 118, which may also aid in system response time.

In some examples, during certain activities engaged by the patient (and the posture of the patient), the pressure in the fluid reservoir 102 may fluctuate. For example, during a period of high activity (e.g., a hard workout) or when the patient is coughing, intra-abdominal pressure may rise, which can lead to incontinence in patients. The pressure spike may be transferred to the fluid reservoir 102. In some examples, if the pressure readings from the pressure sensor 131 is greater than a threshold level, the controller 114 may cause the active valve 118 to transition to the open position so that at least some fluid is transferred to the inflatable member 104 to prevent incontinence caused by increased intra-abdominal pressure.

In some examples, posture may have an effect on intra-abdominal pressure, where standing up may generate a greater pressure than lying down. In some examples, the controller 114 is configured to detect a posture of the patient based on the pressure readings from the pressure sensor 130 and/or the pressure readings from the pressure sensor 131. In some examples, the electronic pump assembly 106 includes an accelerometer (e.g., a single or multi-axis accelerometer) configured to communicate with the controller 114. The accelerometer may detect the acceleration and/or orientation of the body of the patient. The controller 114 may use the readings from the accelerometer to detect the posture or orientation of the patient.

In some examples, if the detected posture of the patient is a position in which the patient is lying down (e.g., a first posture), the controller 114 may reduce the pressure in the inflatable member 104 by activating the pump 120 to transfer a portion of the fluid from the inflatable member 104 to the fluid reservoir 102. If the detected posture of the patient is a position in which the patient is standing up (e.g., a second posture), the controller 114 may increase the pressure in the inflatable member 104 by transitioning the active valve 118 to the open position. In some examples, the controller 114 may detect a change in posture of the patient (e.g., laying down to standing up) based on the pressure readings (e.g., from the pressure sensor 131 and/or the pressure sensor 130), and the controller 114 is configured to adjust the pressure in the inflatable member 104.

In some examples, the electronic pump assembly 106 includes a hermetic enclosure 108 that encloses the components of the electronic pump assembly 106. A hermetic enclosure 108 may be an air-tight (or substantially air-tight) container. The hermetic enclosure 108 may include one or more metal-based materials. In some examples, the hermetic enclosure 108 is a Titanium container. In some examples, the only material in contact with the patient is Titanium. In some examples, the hermetic enclosure 108 includes one or more non-metal-based materials (e.g., ceramic). In some examples, a portion of the hermetic enclosure 108 is a metal-based material and a portion of the hermetic enclosure 108 is a non-metal-based material. In some examples, the hermetic enclosure 108 defines a feedthrough (e.g., a hermetic feedthrough, an electrical feedthrough, a feedthrough connector, etc.) to receive/transmit wireless signals from/to the external device 101. In some examples, the feedthrough includes a metal-based material and an insulator-based material (e.g., ceramic).

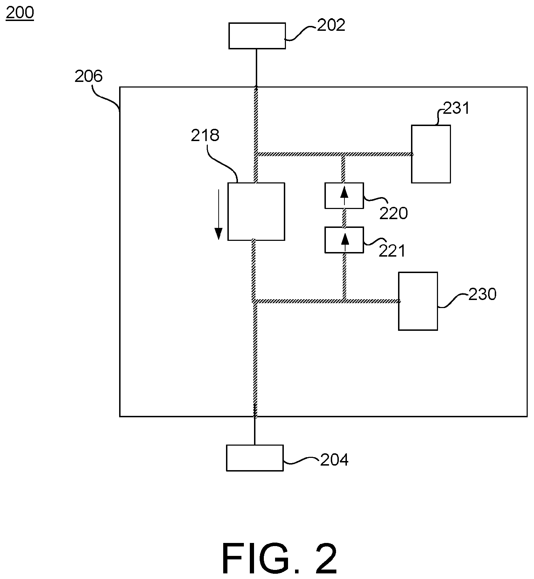

FIG. 2 illustrates an implantable device 200 according to an aspect. The implantable device 200 may be an example of the implantable device 100 of FIG. 1 and may include any of the details discussed with reference to those figures. In some examples, the implantable device 200 is an inflatable penile prosthesis. In some examples, the implantable device 200 is an artificial urinary sphincter device. However, the implantable device 200 may include any type of medical device that transfers fluid between components of the implantable device 200.

The implantable device 200 includes a fluid reservoir 202, an inflatable member 204, and an electronic pump assembly 206 configured to transfer fluid between the fluid reservoir 202 and the inflatable member 204 according to an aspect. The electronic pump assembly 206 may be similar to the electronic pump assembly 106 of FIG. 1 except that the electronic pump assembly 206 includes a pump 220 and a pump 221. The pump 220 and the pump 221 are disposed in series with each other. In some examples, during a deflation cycle, the pump 220 and the pump 221 are activated to transfer fluid back to the fluid reservoir 202. In some examples, the use of serial pumps may decrease the time in which the pressure in the inflatable member 204 can be decreased. The electronic pump assembly 206 also includes an active valve 218. The active valve 218 is in parallel with the pump 220 and the pump 221. The electronic pump assembly 206 includes a pressure sensor 230 configured to monitor the pressure of the inflatable member 204. In some examples, the electronic pump assembly 206 includes a pressure sensor 231 configured to monitor the pressure of the fluid reservoir 202.

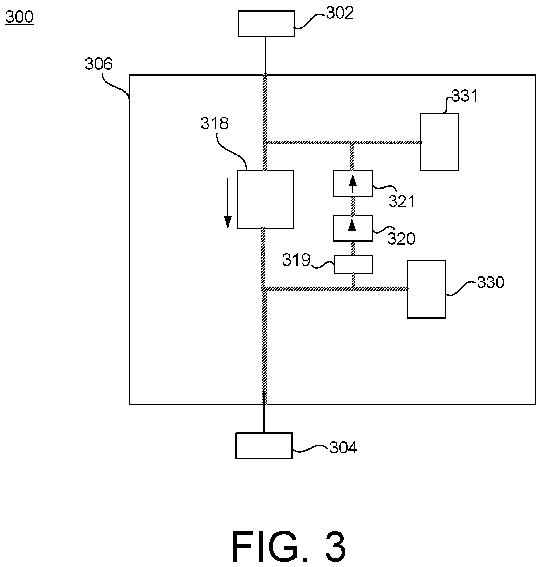

FIG. 3 illustrates an implantable device 300 according to an aspect. The implantable device 300 may be an example of the implantable device 100 of FIG. 1 and/or the implantable device 200 of FIG. 2 and may include any of the details discussed with reference to those figures. In some examples, the implantable device 300 is an inflatable penile prosthesis. In some examples, the implantable device 300 is an artificial urinary sphincter device. However, the implantable device 300 may include any type of medical device that transfers fluid between components of the implantable device 300.

The implantable device 300 includes a fluid reservoir 302, an inflatable member 304, and an electronic pump assembly 306 configured to transfer fluid between the fluid reservoir 302 and the inflatable member 304 according to an aspect. The electronic pump assembly 306 includes a pump 320, a pump 321, and an active valve 319. The pump 320, the pump 321, and the active valve 319 are in series with each other. The electronic pump assembly 306 may include an active valve 318. The active valve 318 may be in parallel in the pump 320, the pump 321, and the active valve 319. The electronic pump assembly 306 includes a pressure sensor 330 configured to monitor the pressure of the inflatable member 304 and a pressure sensor 331 configured to monitor the pressure of the fluid reservoir 302. During an inflation cycle, the active valve 319 may be in the closed position. In some examples, the active valve 319 may reduce leakage through the pump 320 and/or the pump 321. During a deflation cycle, the active valve 319 may be moved to the open position to permit the transfer of fluid into the pump 321 and the pump 320.

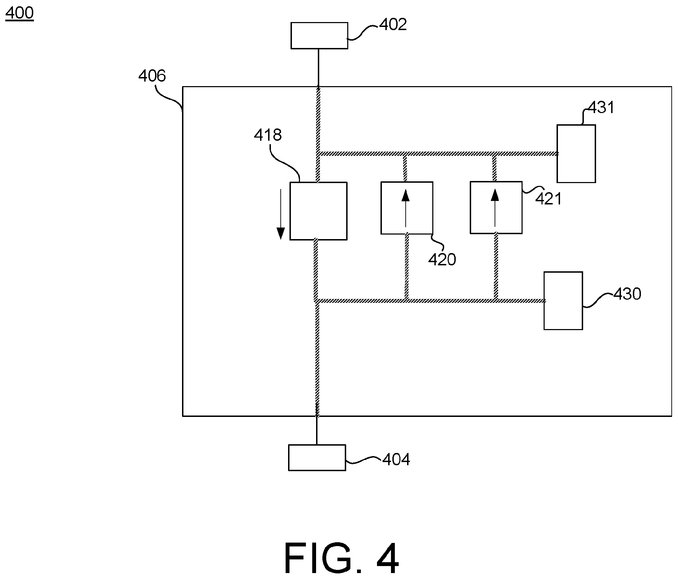

FIG. 4 illustrates an implantable device 400 according to an aspect. The implantable device 400 may be an example of the implantable device 100 of FIG. 1 and may include any of the details discussed with reference to those figures. In some examples, the implantable device 400 is an inflatable penile prosthesis. In some examples, the implantable device 400 is an artificial urinary sphincter device. However, the implantable device 400 may include any type of medical device that transfers fluid between components of the implantable device 400.

The implantable device 400 includes a fluid reservoir 402, an inflatable member 404, and an electronic pump assembly 406 configured to transfer fluid between the fluid reservoir 402 and the inflatable member 404. The electronic pump assembly 406 includes a pump 420 and a pump 421. The pump 421 and the pump 420 are disposed in parallel with each other. The electronic pump assembly 406 includes an active valve 418. The active valve 418 is in parallel with the pump 420 and the pump 421. The electronic pump assembly 406 includes a pressure sensor 430 configured to monitor the pressure of the inflatable member 404 and a pressure sensor 431 configured to monitor the pressure of the fluid reservoir 402.

In some examples, the use of two parallel pumps (e.g., pump 420, pump 421) (or more than two parallel pumps) may increase the amount of fluid that can be transferred from the inflatable member 404 to the fluid reservoir 402. In some examples, the pump 420 and the pump 421 may operate out of phase from each other in order to increase the efficiency of the electronic pump assembly 406. Two parallel pumps (e.g., pump 420, pump 421) operating at out of phase (e.g., 180 degrees of out of phase) from each other may allow the output pressure of the pump 420 to improve the valve closure of the pump 421, thereby improving the overall performance (and vice versa). The use of parallel pumps operating out of phase from each other may allow the pumps to operate at lower frequencies, which can reduce power (thereby extending battery life). Furthermore, a smoother flow rate may also be achieved resulting in less vibration and an improved patient experience. In some examples, the pump 420 and the pump 421 operate at different pumping rates, where one pump transfers fluid into the fluid reservoir 402 at a faster rate than the other pump. For example, pump 420 may operate at a first pumping rate, and pump 421 may operate at a second pumping rate, where the second pumping rate is lower than the first pumping rate.

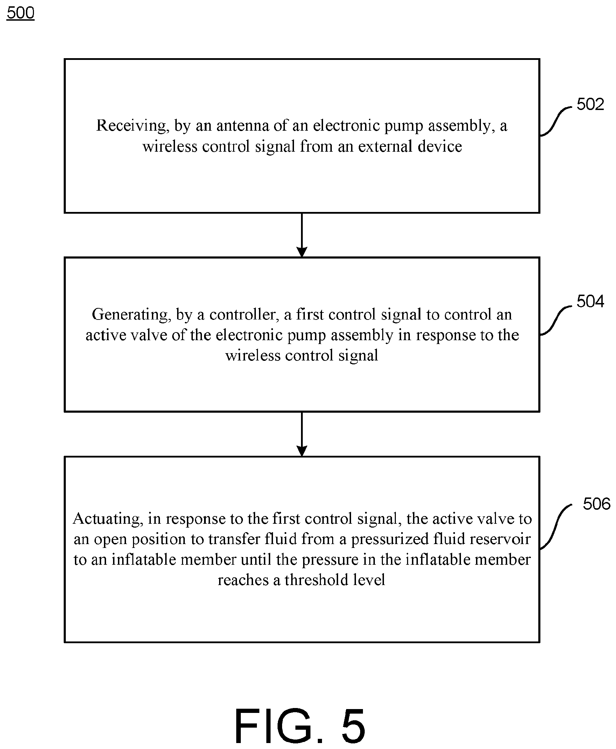

FIG. 5 illustrates a flow chart 500 depicting example operations of a method of transferring fluid in an implantable device according to an aspect. Although the flow chart 500 is explained with reference to the implantable device 100 of FIG. 1, the example operations of the flow chart 500 may be performed by any of the devices discussed herein.

Operation 502 includes receiving, by an antenna 112 of an electronic pump assembly 106, a wireless control signal 109 from an external device 101. Operation 504 includes generating, by a controller 114, a first control signal to control an active valve 118 of the electronic pump assembly 106 in response to the wireless control signal 109. Operation 506 includes actuating, in response to the first control signal, the active valve 118 to an open position to transfer fluid from a pressurized fluid reservoir 102 to an inflatable member 104 until the pressure in the inflatable member 104 reaches a threshold level.

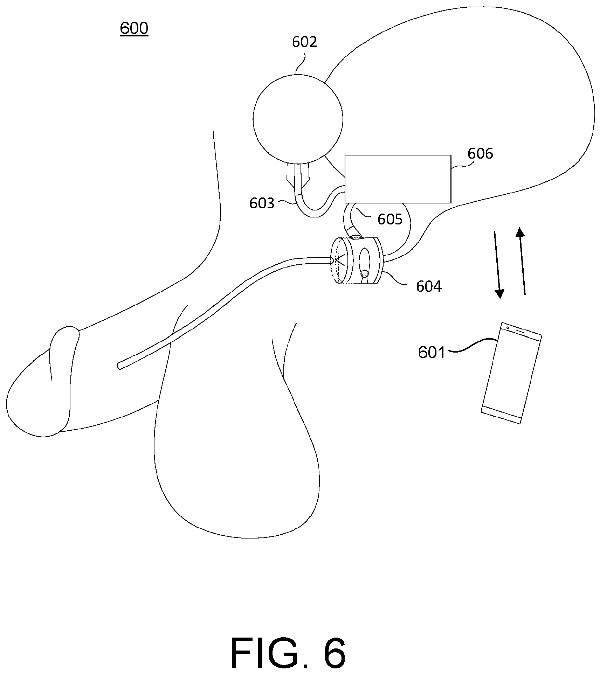

FIG. 6 illustrates a urinary control device 600 having an electronic pump assembly 606 according to an aspect. The electronic pump assembly 606 may include any of the features of the electronic pump assembly (e.g., 106, 206, 306, 406) and the implantable device (e.g., 100, 200, 300, 400) discussed herein. The urinary control device 600 includes a pump assembly 606, a fluid reservoir 602, and a cuff 604.

The fluid reservoir 602 may be a pressure-regulating inflation balloon or element. The fluid reservoir 602 is in operative fluid communication with the cuff 604 via one or more tube members 603, 605. The fluid reservoir 602 is constructed of polymer material that is capable of elastic deformation to reduce fluid volume within the fluid reservoir 602 and push fluid out of the fluid reservoir 602 and into the cuff 604. However, the material of the fluid reservoir 602 can be biased or include a shape memory construct adapted to generally maintain the fluid reservoir 602 in its expanded state with a relatively constant fluid volume and pressure. In some examples, this constant level of pressure exerted from the fluid reservoir 602 to the cuff 604 will keep the cuff 604 at a desired inflated state when open fluid communication is provided between the fluid reservoir 602 and the cuff 604. This is largely due to the fact that only a small level of fluid displacement is required to inflate or deflate the cuff 604. In some examples, the fluid reservoir 602 is implanted into the abdominal space.

A user may use an external device 601 to control the urinary control device 600. In some examples, the user may use the external device 601 to inflate or deflate the cuff 604. For example, in response to the user activating an inflation cycle using the external device 601, the external device 601 may transmit a wireless signal to the electronic pump assembly 606 to initiate the inflation cycle to transfer fluid from the fluid reservoir 602 to the cuff 604 (e.g., by opening an active valve where the pressure in the fluid reservoir 602 causes the fluid to move through the active valve to the cuff 604). In some examples, in response to the user activating a deflation cycle using the external device 601, the external device 601 may transmit a wireless signal to the electronic pump assembly 606 to initiate the deflation cycle to transfer fluid from the cuff 604 to the fluid reservoir 602 using a pump.

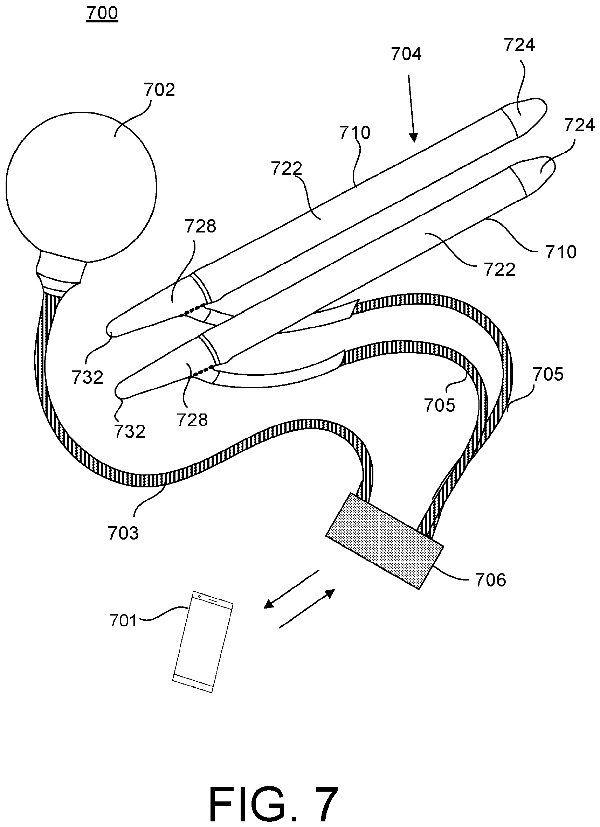

FIG. 7 schematically illustrates an inflatable penile prosthesis 700 having an electronic pump assembly 706 according to an aspect. The electronic pump assembly 706 may include any of the features of the electronic pump assembly (e.g., 106, 206, 306, 406) and the implantable device (e.g., 100, 200, 300, 400) discussed herein. The inflatable penile prosthesis 700 may include an inflatable member 704 (e.g., a pair of inflatable cylinders 710), and the inflatable cylinders 710 are configured to be implanted in a penis. For example, one of the inflatable cylinders 710 may be disposed on one side of the penis, and the other inflatable cylinder 710 may be disposed on the other side of the penis. Each inflatable cylinder 710 may include a first end portion 724, a cavity or inflation chamber 722, and a second end portion 728 having a rear tip 732.

At least a portion of the electronic pump assembly 706 may be implanted in the patient's body. A pair of conduit connectors 705 may attach the electronic pump assembly 706 to the inflatable cylinders 710 such that the electronic pump assembly 706 is in fluid communication with the inflatable cylinders 710. Also, the electronic pump assembly 706 may be in fluid communication with a fluid reservoir 702 via a conduit connector 703. The fluid reservoir 702 may be implanted into the user's abdomen. The inflation chamber 722 of the inflatable cylinder 710 may be disposed within the penis. The first end portion 724 of the inflatable cylinder 710 may be at least partially disposed within the crown portion of the penis. The second end portion 728 may be implanted into the patient's pubic region PR with the rear tip 732 proximate to the pubic bone PB.

In order to implant the inflatable cylinders 710, the surgeon first prepares the patient. The surgeon often makes an incision in the penoscrotal region, e.g., where the base of the penis meets with the top of the scrotum. From the penoscrotal incision, the surgeon may dilate the patient's corpus cavemosum to prepare the patient to receive the inflatable cylinders 710. The corpus cavemosum is one of two parallel columns of erectile tissue forming the dorsal part of the body of the penis, e.g., two slender columns that extend substantially the length of the penis. The surgeon will also dilate two regions of the pubic area to prepare the patient to receive the second end portion 728. The surgeon may measure the length of the corpora cavernosum from the incision and the dilated region of the pubic area to determine an appropriate size of the inflatable cylinders 710 to implant.

After the patient is prepared, the inflatable penile prosthesis 700 is implanted into the patient. The tip of the first end portion 724 of each inflatable cylinder 710 may be attached to a suture. The other end of the suture may be attached to a needle member (e.g., Keith needle). The needle member is inserted into the incision and into the dilated corpus cavernosum. The needle member is then forced through the crown of the penis. The surgeon tugs on the suture to pull the inflatable cylinder 710 into the corpus cavernosum. This is done for each inflatable cylinder 710 of the pair. Once the inflation chamber 722 is in place, the surgeon may remove the suture from the tip. The surgeon then inserts the second end portion 728. The surgeon inserts the rear end of the inflatable cylinder 710 into the incision and forces the second end portion 728 toward the pubic bone PB until each inflatable cylinder 710 is in place.

A user may use an external device 701 to control the inflatable penile prosthesis 700. In some examples, the user may use the external device 701 to inflate or deflate the inflatable cylinders 710. For example, in response to the user activating an inflation cycle using the external device 701, the external device 701 may transmit a wireless signal to the electronic pump assembly 706 to initiate the inflation cycle to transfer fluid from the fluid reservoir 702 to the inflatable cylinders 710. In some examples, in response to the user activating a deflation cycle using the external device 701, the external device 701 may transmit a wireless signal to the electronic pump assembly 706 to initiate the deflation cycle to transfer fluid from the inflatable cylinders 710 to the fluid reservoir 702. In some examples, during the deflation cycle, fluid is transferred back until the pressure in the inflatable cylinders 710 reaches a partial inflation pressure.

Detailed embodiments are disclosed herein. However, it is understood that the disclosed embodiments are merely examples, which may be embodied in various forms. Therefore, specific structural and functional details disclosed herein are not to be interpreted as limiting, but merely as a basis for the claims and as a representative basis for teaching one skilled in the art to variously employ the embodiments in virtually any appropriately detailed structure. Further, the terms and phrases used herein are not intended to be limiting, but to provide an understandable description of the present disclosure.

The terms "a" or "an," as used herein, are defined as one or more than one. The term "another," as used herein, is defined as at least a second or more. The terms "including" and/or "having", as used herein, are defined as comprising (i.e., open transition). The term "coupled" or "moveably coupled," as used herein, is defined as connected, although not necessarily directly and mechanically.

In general, the embodiments are directed to bodily implants. The term patient or user may hereafter be used for a person who benefits from the medical device or the methods disclosed in the present disclosure. For example, the patient can be a person whose body is implanted with the medical device or the method disclosed for operating the medical device by the present disclosure.

## Claims

1. An implantable device (100) comprising:
a pressurized fluid reservoir (102) configured to hold fluid and
an inflatable member (104),
the fluid reservoir (102) being pressurized to a pressure that is greater than a target pressure of the inflatable member (104); and
an electronic pump assembly (106), including:
a pump (120);
an active valve (118);
a pressure sensor (131) configured to monitor a pressure of the inflatable member (104); and
a controller (114) configured to cause the active valve (118) to be in an open position during at least a portion of an inflation cycle to allow the fluid to be transferred from the pressurized fluid reservoir (102) to the inflatable member (104), a pressure differential between the inflatable member (104) and the pressurized fluid reservoir (102) causing the fluid to flow from the fluid reservoir (102) to the inflatable member, the controller (114) configured to transition the active valve (118) to a closed position in response to the pressure of the inflatable member (104) achieving the target pressure.

2. The implantable device (100) of claim 1, wherein the controller (114) is configured to cause the pump (120) to operate during a deflation cycle to transfer the fluid from the inflatable member (104) to the pressurized fluid reservoir (102).

3. The implantable device (100) of claim 2, wherein the electronic pump assembly (106) includes an antenna (112) configured to receive a wireless control signal from an external device (101), the controller (114) configured to activate the inflation cycle or the deflation cycle based on the wireless control signal.

4. The implantable device (100) of any of claims 1 to 3, wherein the fluid is transferred from the pressurized fluid reservoir (102) to the inflatable member (104) during the inflation cycle without using the pump (120).

5. The implantable device (100) of any of claims 1 to 4, wherein the active valve (118) is disposed in parallel with the pump (120).

6. The implantable device (100) of any of claims 1 to 5, wherein the controller (114) is configured to control the pump (120) to operate to transfer a portion of the fluid from the inflatable member (104) to the pressurized fluid reservoir (102) in response to the pressure of the inflatable member (104) being greater than the target pressure.

7. The implantable device (100) of any of claims 1 to 6, wherein the controller (114) is configured to transition the active valve (118) from the open position to a partially-opened position in response to the pressure exceeding a threshold level, the threshold level being a value that is less than the target pressure.

8. The implantable device (100) of any of claims 1 to 7, wherein the pressure sensor (131) is a first pressure sensor, the electronic pump assembly (106) including a second pressure sensor configured to monitor a pressure of the pressurized fluid reservoir (102).

9. The implantable device (100) of claim 8, wherein the controller (114) is configured to cause the active valve (118) to be in the open position for a period of time in response to the pressure of the pressurized fluid reservoir (102) exceeding a threshold level.

10. The implantable device (100) of claim 8, wherein the controller (114) is configured to detect a posture of a patient of the implantable device based on pressure readings from at least one of the first pressure sensor or the second pressure sensor, the controller (114) configured to adjust the pressure of the inflatable member (104) based on the detected posture.

11. The implantable device (100) of any of claims 1 to 10, wherein the pump (120) is a first pump, the electronic pump assembly (106) including a second pump.

12. The implantable device (100) of claim 11, wherein the second pump is in series with the first pump.

## Patentansprüche

1. Implantierbare Vorrichtung (100), umfassend:
einen unter Druck stehenden Fluidbehälter (102), der zur Aufnahme von Fluid eingerichtet ist, und
ein fluidexpandierbares Element (104),
wobei der Fluidbehälter (102) auf einen Druck gebracht wird, der höher ist als ein Zieldruck des fluidexpandierbaren Elements (104); und
eine elektronische Pumpenanordnung (106), die umfasst:
eine Pumpe (120);
ein aktives Ventil (118);
einen Drucksensor (131), der eingerichtet ist, den Druck des fluidexpandierbaren Elements (104) zu überwachen; und
eine Steuereinheit (114), die eingerichtet ist, zu bewirken, dass sich das aktive Ventil (118) während zumindest eines Teils eines Aufblaszyklus in einer offenen Position befindet, um zu ermöglochen, dass das Fluid vom unter Druck stehenden Fluidbehälter (102) zum fluidexpandierbaren Element (104) befördert wird, wobei ein Druckunterschied zwischen dem fluidexpandierbaren Element (104) und dem unter Druck stehenden Fluidbehälter (102) bewirkt, dass das Fluid vom Fluidbehälter (102) zum fluidexpandierbaren Element strömt, wobei die Steuereinheit (114) eingerichtet ist, das aktive Ventil (118) in eine geschlossene Position zu überführen, in Reaktion darauf, dass der Druck des fluidexpandierbaren Elements (104) den Zieldruck erreicht hat.

2. Implantierbare Vorrichtung (100) nach Anspruch 1, wobei die Steuereinheit (114) eingerichtet ist, die Pumpe (120) zu veranlassen, während eines Entleerungszyklus zu arbeiten, um das Fluid aus dem fluidexpandierbaren Element (104) in den unter Druck stehenden Fluidbehälter (102) zu befördern.

3. Implantierbare Vorrichtung (100) nach Anspruch 2, wobei die elektronische Pumpenanordnung (106) eine Antenne (112) umfasst, die eingerichtet ist, ein drahtloses Steuersignal von einer externen Vorrichtung (101) zu empfangen, und wobei die Steuereinheit (114) eingerichtet ist, den Aufblaszyklus oder den Entleerungszyklus auf der Grundlage des drahtlosen Steuersignals zu aktivieren.

4. Implantierbare Vorrichtung (100) nach einem der Ansprüche 1 bis 3, wobei das Fluid während des Aufblaszyklus ohne Einsatz der Pumpe (120) aus dem unter Druck stehenden Fluidbehälter (102) zum fluidexpandierbaren Element (104) befördert wird.

5. Implantierbare Vorrichtung (100) nach einem der Ansprüche 1 bis 4, wobei das aktive Ventil (118) parallel zur Pumpe (120) angeordnet ist.

6. Implantierbare Vorrichtung (100) nach einem der Ansprüche 1 bis 5, wobei die Steuereinheit (114) eingerichtet ist, die Pumpe (120) so steuern, dass diese einen Teil des Fluids aus dem fluidexpandierbaren Element (104) in den Druckfluidbehälter (102) befördert, wenn der Druck des fluidexpandierbaren Elements (104) größer ist als der Zieldruck.

7. Implantierbare Vorrichtung (100) nach einem der Ansprüche 1 bis 6, wobei die Steuereinheit (114) eingerichtet ist, das aktive Ventil (118) als Reaktion darauf, dass der Druck einen Schwellenwert überschreitet, von der geöffneten Position in eine teilweise geöffnete Position zu überführen, wobei der Schwellenwert ein Wert ist, der unter dem Zieldruck liegt.

8. Implantierbare Vorrichtung (100) nach einem der Ansprüche 1 bis 7, wobei der Drucksensor (131) ein erster Drucksensor ist und die elektronische Pumpenanordnung (106) einen zweiten Drucksensor umfasst, der eingerichtet ist, einen Druck des unter Druck stehenden Fluidbehälters (102) zu überwachen.

9. Implantierbare Vorrichtung (100) nach Anspruch 8, wobei die Steuereinheit (114) eingerichtet ist, zu bewirken, dass sich das aktive Ventil (118) für einen bestimmten Zeitraum in der geöffneten Position befindet, wenn der Druck im unter Druck stehenden Fluidbehälter (102) einen Schwellenwert überschreitet.

10. Implantierbare Vorrichtung (100) nach Anspruch 8, wobei die Steuereinheit (114) eingerichtet ist, die Körperhaltung eines Patienten, bei dem die implantierbare Vorrichtung eingesetzt ist, auf der Grundlage von Druckmesswerten mindestens eines der ersten oder zweiten Drucksensoren zu erfassen, und wobei die Steuereinheit (114) eingerichtet ist, den Druck des fluidexpandierbaren Elements (104) entsprechend der erfassten Körperhaltung anzupassen.

11. Implantierbare Vorrichtung (100) nach einem der Ansprüche 1 bis 10, wobei die Pumpe (120) eine erste Pumpe ist und die elektronische Pumpenanordnung (106) eine zweite Pumpe umfasst.

12. Implantierbare Vorrichtung (100) nach Anspruch 11, wobei die zweite Pumpe in Reihe mit der ersten Pumpe angeordnet ist.

## Revendications

1. Dispositif implantable (100) comprenant :
un réservoir de fluide sous pression (102) configuré pour contenir du fluide et
un élément gonflable (104),
le réservoir de fluide (102) étant mis sous pression à une pression qui est supérieure à une pression cible de l'élément gonflable (104) ; et
un ensemble de pompes électroniques (106), incluant :
une pompe (120) ;
une soupape active (118) ;
un capteur de pression (131) configuré pour surveiller une pression de l'élément gonflable (104) ; et
un contrôleur (114) configuré pour forcer la soupape active (118) à être dans une position ouverte pendant au moins une partie d'un cycle de gonflage afin de permettre le transfert du fluide depuis le réservoir de fluide sous pression (102) jusqu'à l'élément gonflable (104), un différentiel de pression entre l'élément gonflable (104) et le réservoir de fluide sous pression (102) forçant le fluide à s'écouler depuis le réservoir de fluide (102) jusqu'à l'élément gonflable, le contrôleur (114) étant configuré pour faire en sorte que la soupape active (118) effectue une transition jusqu'à une position fermée en réponse au fait que la pression de l'élément gonflable (104) a atteint la pression cible.

2. Dispositif implantable (100) selon la revendication 1, dans lequel le contrôleur (114) est configuré pour forcer la pompe (120) à fonctionner pendant un cycle de dégonflage afin de transférer le fluide depuis l'élément gonflable (104) jusqu'au réservoir de fluide sous pression (102).

3. Dispositif implantable (100) selon la revendication 2, dans lequel l'ensemble de pompes électroniques (106) inclut une antenne (112) configurée pour recevoir un signal de commande sans fil en provenance d'un dispositif externe (101), le contrôleur (114) étant configuré pour activer le cycle de gonflage ou le cycle de dégonflage sur la base du signal de commande sans fil.

4. Dispositif implantable (100) selon l'une quelconque des revendications 1 à 3, dans lequel le fluide est transféré depuis le réservoir de fluide sous pression (102) jusqu'à l'élément gonflable (104) pendant le cycle de gonflage sans utiliser la pompe (120).

5. Dispositif implantable (100) selon l'une quelconque des revendications 1 à 4, dans lequel la soupape active (118) est disposée en parallèle à la pompe (120).

6. Dispositif implantable (100) selon l'une quelconque des revendications 1 à 5, dans lequel le contrôleur (114) est configuré pour commander la pompe (120) afin qu'elle fonctionne pour transférer une partie du fluide depuis l'élément gonflable (104) jusqu'au réservoir de fluide sous pression (102) en réponse au fait que la pression de l'élément gonflable (104) est supérieure à la pression cible.

7. Dispositif implantable (100) selon l'une quelconque des revendications 1 à 6, dans lequel le contrôleur (114) est configuré pour faire en sorte que la soupape active (118) effectue une transition depuis la position ouverte jusqu'à une position partiellement ouverte en réponse au fait que la pression excède un niveau de seuil, le niveau de seuil étant une valeur qui est inférieure à la pression cible.

8. Dispositif implantable (100) selon l'une quelconque des revendications 1 à 7, dans lequel le capteur de pression (131) est un premier capteur de pression, l'ensemble de pompes électroniques (106) incluant un second capteur de pression configuré pour surveiller une pression du réservoir de fluide sous pression (102).

9. Dispositif implantable (100) selon la revendication 8, dans lequel le contrôleur (114) est configuré pour forcer la soupape active (118) à être dans la position ouverte pendant une période de temps en réponse au fait que la pression du réservoir de fluide sous pression (102) excède un niveau de seuil.

10. Dispositif implantable (100) selon la revendication 8, dans lequel le contrôleur (114) est configuré pour détecter une posture d'un patient porteur du dispositif implantable sur la base de lectures de pression en provenance d'au moins un capteur de pression parmi le premier capteur de pression et le second capteur de pression, le contrôleur (114) étant configuré pour régler la pression de l'élément gonflable (104) sur la base de la posture détectée.

11. Dispositif implantable (100) selon l'une quelconque des revendications 1 à 10, dans lequel la pompe (120) est une première pompe, l'ensemble de pompes électroniques (106) incluant une seconde pompe.

12. Dispositif implantable (100) selon la revendication 11, dans lequel la seconde pompe est en série avec la première pompe.
